# EUROPÄISCHE PATENTSCHRIFT

(11) **EP 1 994 071 B1**
(45) Veröffentlichungstag und Bekanntmachung des Hinweises auf die Patenterteilung: **26.05.2010**
(21) Anmeldenummer: 07726849.8
(22) Anmeldetag: 13.03.2007
(51) Int. Cl.: C08G 63/00, C08G 65/00

(54) **FORMGEDÄCHTNISPOLYMER MIT POLYESTER- UND POLYETHERSEGMENTEN UND VERFAHREN ZU SEINER HERSTELLUNG UND PROGRAMMIERUNG**
SHAPE MEMORY POLYMER WITH POLYESTER AND POLYETHER SEGMENTS AND PROCESS FOR ITS PREPARATION AND PROGRAMMING
POLYMERE A MEMOIRE DE FORME COMPRENANT DES SEGMENTS POLYESTER ET POLYETHER, SON PROCEDE DE FABRICATION ET SA PROGRAMMATION

(30) Priorität: 14.03.2006 DE 102006012169
(43) Veröffentlichungstag der Anmeldung: 26.11.2008
(73) Patentinhaber: GKSS-Forschungszentrum Geesthacht GmbH, 21502 Geesthacht (DE)
(72) Erfinder: KELCH, Steffen, 8102 Oberengstringen (CH); LENDLEIN, Andreas, 14167 Berlin (DE); BELLIN, Ingo, 68159 Mannheim (DE)
(74) Vertreter: Reinstädler, Diane
(86) Internationale Anmeldenummer: PCT/EP2007/052345
(87) Internationale Veröffentlichungsnummer: WO 2007/104757

(56) Entgegenhaltungen:
- EP-A- 1 362 879
- WO-A-2004/046221
- WO-A-2004/090042

## Beschreibung

Die Erfindung betrifft ein Formgedächtnispolymer, das - neben einer permanenten Form - mindestens zwei temporäre Formen speichern kann, ein Verfahren zu seiner Herstellung und ein Verfahren zu seiner Formprogrammierung sowie seine Verwendung.

Im Stand der Technik sind so genannte Formgedächtnispolymere oder SMPs (shape memory polymers) bekannt, die bei Induktion durch einen geeigneten Stimulus einen Formübergang von einer temporären Form in eine permanente Form entsprechend einer vorherigen Programmierung zeigen. Am häufigsten ist dieser Formgedächtniseffekt thermisch stimuliert, das heißt bei Erwärmung des Polymermaterials über die definierte Übergangstemperatur findet die durch Entropieelastizität angetriebene Rückstellung statt. Formgedächtnispolymere sind in der Regel Polymernetzwerke, bei denen chemische (kovalente) oder physikalische (nicht kovalente) Vernetzungsstellen die permanente Form bestimmen. Die Programmierung erfolgt, indem oberhalb der Übergangstemperatur eines Schaltsegmentes das Polymermaterial deformiert und anschließend unter Aufrechterhaltung der Deformationskräfte unter diese Temperatur abgekühlt wird, um die temporäre Form zu fixieren. Erneute Erwärmung oberhalb der Übergangstemperatur führt zu einem Phasenübergang und Wiederherstellung der ursprünglichen permanenten Form.

Darüber hinaus sind in jüngerer Zeit auch Polymernetzwerke beschrieben worden, die zwei Schaltsegmente mit unterschiedlichen Übergangstemperaturen aufweisen.

So beschreibt EP 1 362 879 A Formgedächtnispolymere (in diesem Fall interpenetrierende Netzwerke IPNs), die aus einer kovalent vernetzten Polymerkomponente, insbesondere auf Basis von Caprolacton-, Lactid-, Glycolid- oder p-Dioxanoneinheiten, und einer nicht kovalent vernetzten Polyesterurethankomponente bestehen. Das Polymer kann zwei temporäre Formen speichern, wobei Übergangstemperaturen um 50 und 90°C beschrieben werden.

Auch aus Liu et al. (Macromol. Rap. Comm. 26, 2005, 649ff) ist ein SMP (semi-interpenetrierendes Netzwerk SIPN) bekannt, bestehend aus Polymethylmethacrylateinheiten (PMMA) und Polyethylenglycoleinheiten (PEG), das ebenfalls zwei Übergangstemperaturen (40 und 86°C) aufweist. Das dort beschriebene Programmierverfahren erlaubt jedoch nur die Speicherung einer temporären Form.

WO 2004/046221 A beschreibt ein Formgedächtnispolymer, das durch Vernetzen eines ABA-Triblockdimethacrylats als Makromonomer hergestellt wird. Dabei handelt es sich bei den Blöcken des ABA-Triblockpolymers um Polyester und Polyether. Durch die Vernetzung der ABA-Dimethacylate entstehen kovalent vernetzte dreidimensionale Netzwerke, bei denen sämtliche Blöcke beidseitig gebunden vorliegen. Das Material besitzt Zweiformeneigenschaften, d.h. es kann nach einer thermomechanischen Programmierung einen Formenübergang vollziehen.

Aus WO 2004/090042 A ist ein Polymerblend zweier Multiblockcopolymere bekannt, welche jeweils ein Hart- und ein Weichsegment umfassen, wobei die Weichsegemente identisch sind. Als bevorzugte Hartsegmente werden Poly(p-dioxanon) und Poly(ε-caprolacton) genannt und als Weichsegemente Poly(ε-caprolacton-co-glycolid), ein Polyester, ein Polyetherester, insbesondere Polyalkenylenadipinat. Die Multiblockcoppolymere selbst zeigen keinen Formgedächtniseffekt, jedoch der Blend, welcher Zweiformeneigenschaften aufweist.

Ein wichtiges Einsatzgebiet für Formgedächtnispolymere ist die Medizintechnik, wo derartige Materialien beispielsweise als selbst verknotendes Nahtmaterial oder Implantatmaterial eingesetzt werden können. Für viele derartige Anwendungen sind resorbierbare Polymere wünschenswert, die nach einiger Zeit hydrolytisch im Körper abgebaut werden. Nachteilig an den bekannten Formgedächtnispolymeren ist, dass ihre Schalttemperaturen außerhalb eines physiologisch verträglichen Bereichs liegen, insbesondere zu hoch sind, und/oder dass sie nicht resorbierbar bzw. sie oder ihre Abbauprodukte nicht biokompatibel sind.

Der Erfindung liegt die Aufgabe zugrunde, ein neues biokompatibles Formgedächtnispolymer bereitzustellen, das zumindest zwei temporäre Formen speichern kann. Die entsprechenden Schalttemperaturen des Polymers sollen insbesondere in einem physiologisch akzeptablen Bereich liegen, das heißt die Stimulation des Formgedächtnisses soll möglichst ohne Schädigung umgebender Zellen möglich sein. Des Weiteren soll ein Verfahren zur Programmierung von zumindest zwei temporären Formen des Formgedächtnispolymers zur Verfügung gestellt werden.

Diese Aufgabe wird gelöst durch ein Formgedächtnispolymer mit den Merkmalen des Anspruchs 1. Das erfindungsgemäße Formgedächtnispolymer weist mindestens zwei Schaltsegmente mit unterschiedlichen Übergangstemperaturen auf, sodass das Polymermaterial in Abhängigkeit von der Temperatur neben einer permanenten Form mindestens zwei temporäre Formen einnehmen kann. Das erfindungsgemäße Polymersystem umfasst ein erstes Schaltsegment, das im Wesentlichen auf einem Polyester der allgemeinen Formel I mit n = 1...6 oder einem Derivat von diesem basiert oder auf einem Co-Polyester der allgemeinen Formel I mit n = 1...6, worin mindestens zwei Estereinheiten mit unterschiedlichen Kettenlängen n vorhanden sind, oder einem Derivat von diesem.

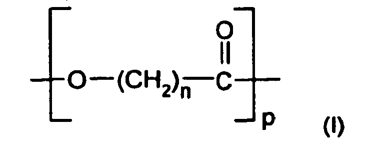

Das Polymersystem umfasst ferner ein zweites Schaltsegment, das im Wesentlichen auf einem Polyether der allgemeinen Formel II mit m = 1...4 basiert oder auf einem Co-Polyether der allgemeinen Formel II mit m = 1...4, worin mindestens zwei Ethereinheiten mit unterschiedlichen Kettenlängen m vorhanden sind, oder einem Derivat von diesen.

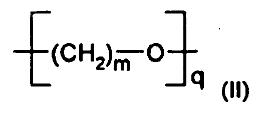

Im erfindungsgemäßen Formgedächtnispolymer liegt eines der Schaltsegmente vernetzt vor und das andere Schaltsegment in Form freier Seitenketten, die an dem vernetzten Schaltsegment oder an einer polymeren Rückgratstruktur (einseitig) gebunden sind.

Dabei wird unter dem Begriff Schaltsegment ein Oligomer oder Polymer nach den angegebenen Formeln I bzw. II verstanden, das eine Kettenlänge p bzw. q aufweist, welche die Ausbildung einer eigenen Phase durch Phasenentmischung im Festkörper und damit die Grundlage zur Ausbildung der typischen Materialeigenschaften der entsprechenden Verbindung gestattet. Auf diese Weise wird erreicht, dass das Polymersystem als Ganzes Materialeigenschaften aufweist, die den jeweiligen Schaltsegmenten zugeordnet werden können, insbesondere zwei oder mehrere unterschiedliche Schalttemperaturen für den thermisch induzierten Effekt, bei denen es sich unabhängig voneinander um Glasübergangs- oder Schmelztemperaturen handeln kann. In struktureller Hinsicht können die Schaltsegmente kovalent oder nicht kovalent vernetzt vorliegen und endständig, einseitig oder beidseitig miteinander und/oder mit einem Polymerrückgrat verknüpft sein. Des Weiteren umfassen im Rahmen der vorliegenden Erfindung Derivate des Polyesters nach Formel I und/oder Derivate des Polyethers nach Formel II Strukturen, in denen einer oder mehrere der Wasserstoffreste der Methyleneinheiten (-CH₂-) durch unverzweigte oder verzweigte, gesättigte oder ungesättigte C1- bis C6-Reste ausgetauscht sind. Entscheidend bei der Auswahl der Substituenten im angegebenen Rahmen ist, dass die Ausbildung einer eigenen Phase der Schaltsegmente nicht verhindert wird.

Durch die erfindungsgemäße Zusammensetzung wird ein Material zur Verfügung gestellt, das nach entsprechender Programmierung in der Lage ist, zumindest zwei Deformationen gleichzeitig zu fixieren, die nach Aktivierung durch entsprechende thermische Stimuli wieder hergestellt werden können. Als eine besonders vorteilhafte Eigenschaft des erfindungsgemäßen Polymersystems haben sich Schalttemperaturen herausgestellt, die in einem physiologisch akzeptablen Bereich liegen. Insbesondere liegen die beiden Schalttemperaturen der Schaltsegmente nach Formel I und II unterhalb von 85°C. Bevorzugt werden die Monomereinheiten, ihre Substituenten sowie die Kettenlängen der Schaltsegmente so gewählt, dass die Schalttemperaturen unterhalb von 80°C, vorzugsweise unterhalb von 75°C, liegen. Ein weiterer Vorteil besteht darin, dass beide Schaltsegmentpolymere physiologisch resorbierbar sind und ihre Abbauprodukte physiologisch kompatibel sind.

In bevorzugter Ausgestaltung der Erfindung umfasst das erste Schaltsegment ein Poly(ε-caprolacton)-Segment mit n = 5 oder ein Derivat von diesem, in dem die aliphatischen Kohlenstoffatome unabhängig voneinander mit einem oder zwei, unverzweigten oder verzweigten, gesättigten oder ungesättigten C1- bis C6-Resten substituiert sein können. Besonders bevorzugt ist jedoch nicht derivatisiertes Poly(ε-caprolacton) mit n = 5 nach Formel I, das heißt ohne Substituenten.

In weiterer vorteilhafter Ausgestaltung der Erfindung umfasst das zweite Schaltsegment ein Polyethylenglycol-Segment mit m = 2 oder ein Derivat von diesem, in dem die aliphatischen Kohlenstoffatome unabhängig voneinander mit einem oder zwei, unverzweigten oder verzweigten, gesättigten oder ungesättigten C1- bis C6-Resten substituiert sein können. Besonders bevorzugt ist jedoch wiederum nicht derivatisiertes Polyethylenglycol mit m = 2 nach Formel II.

Die Molekulargewichte der Segmente sowie ihre Massenanteile im Polymer und ihre relativen Massenverhältnisse (erstes Schaltsegment : zweites Schaltsegment) sind so abgestimmt, dass die oben beschriebenen Schalttemperaturen nicht überschritten werden und deutliche Formveränderungen bei den zumindest zwei Phasenübergängen erzielt werden. Mit Vorteil weist das erste Schaltsegment (Polyester) ein mittleres Molekulargewicht im Bereich von 2.000 bis 100.000 g/mol, insbesondere von 5.000 bis 40.000 g/mol, vorzugsweise von etwa 10.000 g/mol auf. Unabhängig davon haben sich mittlere Molekulargewichte des zweiten Schaltsegments (Polyether) im Bereich von 100 bis 10.000 g/mol bewährt, insbesondere von 500 bis 5.000 g/mol, vorzugsweise von etwa 1.000 g/mol. Vorzugsweise liegt ein Massenanteil des Polyestersegments im Formgedächtnispolymer im Bereich von 25 bis 65 %, insbesondere im Bereich von 30 bis 60 %. Entsprechend weist das Polyethersegment einen Massenanteil im Bereich von 35 bis 75 %, insbesondere im Bereich von 40 bis 70 %, auf.

Bei dem erfindungsgemäßen Polymersystem handelt es sich um ein Polymernetzwerk, in dem die die Schaltsegmente aufweisenden Polymerketten miteinander vernetzt vorliegen. Vorzugsweise liegt es als ein Polymernetzwerk vor, in dem der Polyester vernetzt vorliegt und der Polyether als freie Seitenketten, die an dem vernetzten Polyester oder einer polymeren Rückgratstruktur gebunden sind. Dabei kann gemäß einer speziellen Ausgestaltung der Erfindung die Rückgratstruktur durch die vernetzenden Einheiten beider Polymerkomponenten selbst gebildet werden, insbesondere durch Acrylat- oder Methacrylatendgruppen.

Das erfindungsgemäße Formgedächtnispolymer kann vorteilhaft durch ein Verfahren hergestellt werden, indem
- ein Polyester-Makromer der allgemeinen Formel I a, worin n = 1...6 und Y ein beliebiger verbindender Rest ist, oder einem Co-Polyester der allgemeinen Formel I a (worin n und Y die obige Bedeutung haben) mit mindestens zwei Estereinheiten mit unterschiedlichen n oder einem Derivat von diesen und 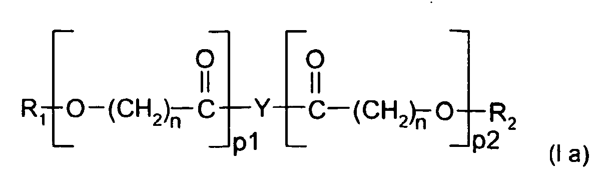
- ein Polyether-Makromonomer der allgemeinen Formel II a mit m = 1...4 oder einem Co-Polyether der allgemeinen Formel II a mit mindestens zwei Ethereinheiten mit unterschiedlichen m oder einem Derivat von diesen 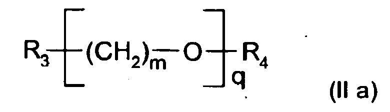 miteinander copolymerisiert werden. Bevorzugte Ausgestaltungen des Polyesters und des Polyethers werden entsprechend der vorstehenden Beschreibung gewählt. Dabei können in Formel I a p1 und p2, das heißt die Kettenlängen der Polyester bzw. Co-Polyester, gleich oder ungleich sein. Der Rest Y dient ausschließlich der Verbindung der beiden Polyestereinheiten unter Umkehrung der Kettenrichtung, sodass beidseitig polymerisationsfähige Endgruppen angefügt werden können, die der Vernetzung dienen (s.u.).

Ein geeignetes Makromer der Polyesterkomponente entspricht beispielsweise der allgemeinen Formel I b mit r = 2...8 und X = O oder NH. Besonders bevorzugt ist eine Komponente mit r = 2, p3 = 2 und X=O, das heißt das Polyester-Makromer wird durch Polymerisation von Diethylenglycol HO-CH₂-CH₂-O-CH₂-CH₂-OH mit den entsprechenden Estermonomeren erhalten.

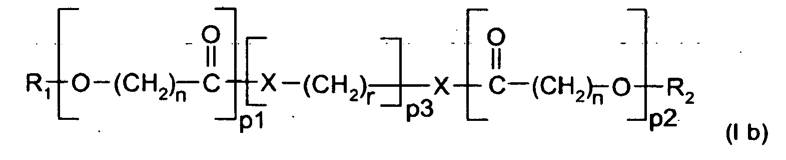

Bevorzugt sind die erste Endgruppe R₁ u nd/oder die zweite Endgruppe R₂ des ersten Schaltsegments unabhängig voneinander ein polymerisierbarer Rest. Vorzugsweise sind sowohl R₁ als auch R₂ jeweils ein polymerisierbarer Rest. Besonders bevorzugt werden für R₁ und/oder R₂ Acryl- oder Methacrylreste eingesetzt, insbesondere jeweils ein Methacrylrest. Auf diese Weise erhält man bei der Copolymerisation beider Komponenten ein Netzwerk, in dem die Polyestersegmente beidseitig verknüpft sind, wobei durch Polymerisation der Polyesterkomponente untereinander Poly(meth)acrylatketten gebildet werden, die ein Polymerrückgrat bilden, welches durch die Polyesterketten querernetzt ist.

Gleichfalls können die erste Endgruppe R₃ und/oder die zweite Endgruppe R₄ des zweiten Schaltsegments unabhängig voneinander ein polymerisierbarer Rest sein. Bevorzugt ist nur eine der Endgruppen R₃ oder R₄ ein polymerisierbarer Rest und die andere ein nicht reaktiver Rest. Diese Maßnahme führt zu einer nur einseitig stattfindenden Anknüpfung (Pfropfung) des entsprechenden Schaltsegmentes entweder an das andere Schaltsegment oder an die optional vorhandene Rückgratstruktur. Nach einer besonders bevorzugten Ausführung ist die erste Endgruppe R₃ oder die zweite Endgruppe R₄ ein Acryl- oder ein Methacrylrest und die andere Endgruppe ein Alkoxyrest, wobei insbesondere die erste Endgruppe R₃ ein Methyletherrest und die zweite Endgruppe R₄ ein Methacrylatrest ist.

Des Weiteren kann die Copolymerisation in Gegenwart zumindest eines weiteren Monomeren durchgeführt werden, insbesondere in Gegenwart eines Acryl- oder Methacrylmonomers. Somit wird ein Poly(meth)acrylat gebildet, das eine zusätzliche Komponente der oben erwähnten Rückgratstruktur ausbildet.

In einer besonders bevorzugten Ausgestaltung wird somit als erstes Makromer Poly(ε-caprolacton)-dimethacrylat (PCLDMA) nach Formel I c mit dem zweiten Makromer Polyethylenglycolmethylethermethacrylat (PEGMMA) nach Formel II c copolymerisiert. Hierdurch entsteht eine Rückgratstruktur, die aus linear polymerisierten Methacrylateinheiten aus PCLDMA-Makromeren und PEGMMA-Makromeren besteht. Dieses Rückgrat wird durch (beidseitig gebundene) PCL-Ketten verknüpft und trägt PEG-Ketten in Form (einseitig gebundener) freier Seitenketten.

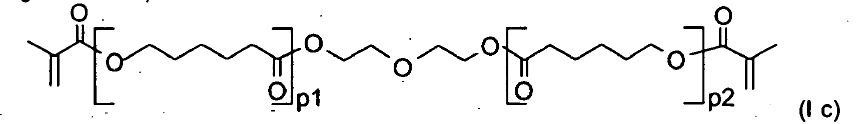

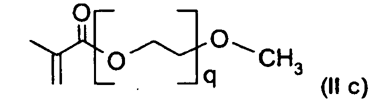

Ein weiterer wichtiger Aspekt der Erfindung betrifft ein Verfahren zur Programmierung von mindestens zwei temporären Formen bei einem Formgedächtnispolymer gemäß der Erfindung. Das erfindungsgemäße Verfahren umfasst die Schritte
(a) Überführung des Formgedächtnispolymers in eine der ersten temporären Form entsprechende Form bei einer Temperatur oberhalb der oberen Übergangstemperatur,
(b) Abkühlung auf eine Temperatur unterhalb der oberen Übergangstemperatur unter Fixierung der ersten temporären Form,
(c) Überführung des Formgedächtnispolymers in eine der zweiten temporären Form entsprechende Form bei einer Temperatur oberhalb der unteren Übergangstemperatur und unterhalb der oberen Übergangstemperatur und
(d) Abkühlung auf eine Temperatur unterhalb der unteren Übergangstemperatur unter Fixierung der zweiten temporären Form.

Dabei kann die in Schritt (b) erfolgende Abkühlung wahlweise auf eine Zwischentemperatur unterhalb der oberen Übergangstemperatur und oberhalb der unteren Übergangstemperatur abgekühlt werden oder aber auf eine Temperatur unterhalb der unteren Übergangstemperatur. Entscheidend für die Fixierung der ersten temporären Form ist, dass unterhalb der oberen Übergangstemperatur abgekühlt wird. Handelt es sich bei dem Formgedächtnispolymer um eines, das mehr als zwei temporäre Formen speichern kann, das heißt zumindest drei Schaltsegmente aufweist, werden die weiteren temporären Formen in analoger Weise programmiert, indem jeweils oberhalb der entsprechenden Übergangstemperatur eine Deformationskraft ausgeübt wird und die temporäre Form durch Kühlung unterhalb dieser Übergangstemperatur unter Beibehaltung der Deformationskraft fixiert wird.

Das erfindungsgemäße Formgedächtnispolymer eignet sich besonders vorteilhaft für medizinische Anwendungen, insbesondere als Implantatmaterial. Beispielsweise kann es als intelligentes Implantatmaterial verwendet werden, wobei durch thermische Stimulation die gespeicherten Formen abgerufen werden und hierdurch eine Anpassung an die anatomischen Gegebenheiten möglich wird. Dabei kommen vorteilhaft die physiologisch unbedenklichen Übergangstemperaturen sowie die gute Bioresorption zum Tragen.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen erläutert. Es zeigen:
- Figur 1: Struktur eines erfindungsgemäßen Pfropfpolymernetzwerkes, erhalten durch Copolymerisation von PCLDMA und PEGMMA,
- Figur 2: DSC- und DMTA-Untersuchungen der Phasenübergänge von PCL-PEG- Netzwerken unterschiedlicher Zusammensetzungen,
- Figur 3: Programmierung eines Pfropfpolymemetzwerkes gemäß Figur 1,
- Figur 4: zeitliche Verläufe verschiedener Programmierungsparameter in einem zy- klischen, thermomechanischen Experiment und
- Figur 5 -: Formgedächtnispolymer gemäß der Erfindung in einem Anwendungsbei- spiel.

### 1. Synthese von Poly(ε-caprolacton)dimethacrylat PCL10kDMA

500 g (50 mmol) Poly(ε-caprolacton)diol (Aldrich) mit einem mittleren Molekulargewicht von 10 000 g/mol (PCL10k-diol) wurden in 5 I Dichlormethan in einem trockenen 3-Halskolben unter Stickstoffatmosphäre vorgelegt. Unter Eiskühlung wurden 20,0 ml (0,14 mol) Triethylamin tropfenweise zugegeben. Nach 10 min Rühren bei 0°C wurden 17,4 ml (0,18 mol) Methacryloylchlorid tropfenweise zugegeben. Die Lösung wurde auf RT erwärmt und für weitere 24 h gerührt. Das ausgefallene Salz wurde durch Filtration entfernt. Das Filtrat wurde aufkonzentriert und in Ethylacetat gelöst. Diese Lösung wurde in einem 10fachen Überschuss einer Mischung aus Hexan/Diethylether/Methanol (18:1:1 Volumenanteile) bei - 20°C ausgefällt. Nach Vakuumtrocknung wurden 475 g (47 mmol) Poly(ε-caprolacton)dimethacrylat PCLDMA mit einem mittleren Molekulargewicht von 10 kD (PCL10kDMA) nach der Formel I c (s.o.) erhalten (Ausbeute 95%). Der Funktionalisierungsgrad der PCL-diole mit Methacrylatendgruppen wurde mit ¹H-NMR-Spektroskopie auf ca. 85% bestimmt. Dies bedeutet, dass 72% der Makromere beidseitig funktionalisiert wurden (Dimethacrylat), 26% einseitig funktionalisiert wurden (Monomethacrylat) und 2% nicht funktionalisiert als Diol vorlagen.

### 2. Copolymerisation von PCLDMA und PEGMMA

PCL10kDMA hergestellt nach Beispiel 1 und Polyethylenglycolmethylethermethacrylat (PEG1kMMA) (Polyscience) gemäß Formel II c (s.o.) mit einem mittleren Molekulargewicht von 1.000 g/mol wurden in verschiedenen Mischungsverhältnissen gemäß Tabelle 1 eingewogen. (Der Funktionalisierungsgrad mit Methylmethacrylat(MMA)-Endgruppen von PEG1kMMA wurde mittels MALDI-TOF Massenspektrometrie auf 100% bestimmt.) Die Mischungen aus PCL10kDMA und PEG1kMMA wurden bei 80°C im Vakuumofen geschmolzen, um eine gute Vermischung zu erhalten. Diese Vorpolymermischung wurde auf eine Glasplatte (10 x 10 cm) ausgegossen und wieder in den Ofen gestellt. Nach Vorliegen einer blasenfreien homogenen Schmelze wurde die Form durch eine aufgelegte weitere Glasplatte unter seitlicher Anordnung von PTFE-Abstandhaltem (Dicke 0,55 cm) geschlossen. Das durch Klammern fixierte Gebilde wurde für 80 min UV-bestrahlt (Fe-dotierte Quecksilberdampflampe), um die Vernetzung auszulösen. Als Vergleichsmaterialien wurde reines PCL10kDMA und reines PEG1kMMA entsprechend behandelt, um ein Homopolymernetzwerk aus PCL10kDMA zu erhalten (PCL(100) in Tabelle 1) bzw. lineare Homopolymere aus PEG1kMMA (P[PEGMMA] in Tabelle 1).

**Tabelle 1**

| Polymer^{#} | PCL10kDMA [g] | PEG1kMMA [g] |
|---|---|---|
| P[PEGMMA] | - | 8,50 |
| PCL(20)PEG | 1,50 | 6,00 |
| PCL(30)PEG | 2,25 | 5,25 |
| PCL(40)PEG | 3,00 | 4,50 |
| PCL(50)PEG | 3,50 | 3,50 |
| PCL(60)PEG | 4,53 | 3,02 |
| PCL(70)PEG | 5,25 | 2,25 |
| PCL(80)PEG | 6,00 | 1,50 |
| PCL(100) | 8,50 | - |

| | | |
|---|---|---|
| ^{#} Die in den Klammern angegebenen Zahlen bezeichnen den Massenanteil an PCL10RDMA im Polymernetzwerk. | | |

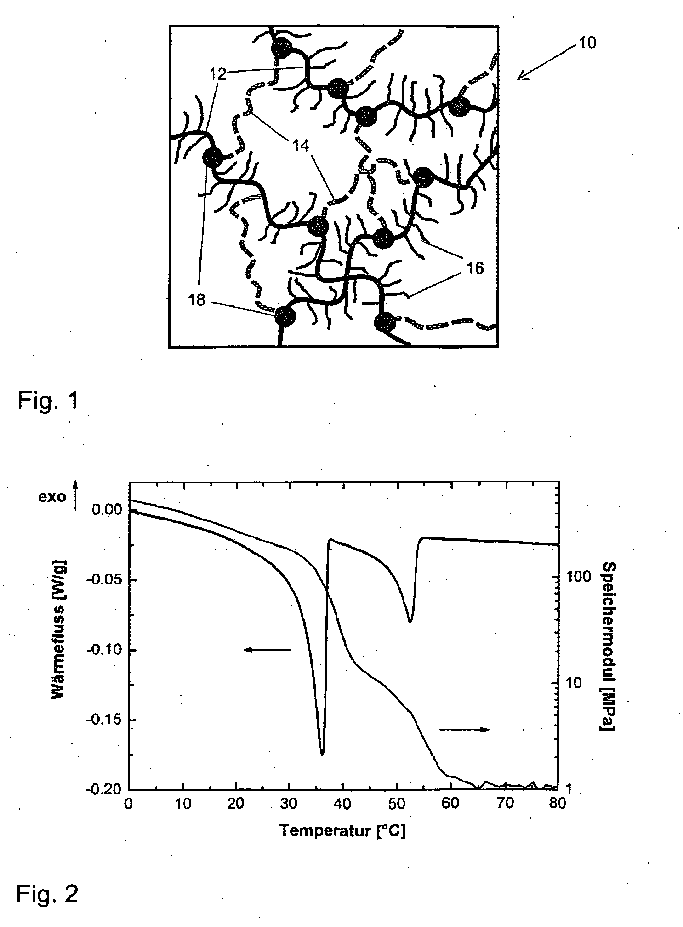

Figur 1 zeigt schematisch die Struktur eines so erhaltenen insgesamt mit 10 bezeichneten PCL-PEG-Polymernetzwerkes. Hierin ist das Rückgrat mit 12 bezeichnet. Das Rückgrat 12 setzt sich im Wesentlichen aus linearen Polymethacrylatketten der miteinander polymerisierten PCL10kDMA- und PEG1kMMA-Makromere zusammen. Das Rückgrat 12 wird durch beidseitig gebundene PCL10kDMA-Ketten 14 vernetzt, während PEG1kMMA 16 als freie Seitenketten am Rückgrat 12 gebunden vorliegen. Mit 18 sind die Verknüpfungspunkte bezeichnet, welche die Verbindungsstellen zwischen dem Rückgrat 12 und den PCL10kDMA-Ketten 14 darstellen.

### 3. Charakterisierung der Polymernetzwerke aus PCLDMA und PEGMMA

Die thermischen Eigenschaften der laut Beispiel 2 hergestellten Polymernetzwerke aus PCL10kDMA- und PEG1kMMA-Makromeren unterschiedlicher Zusammensetzung wurden mit Dynamischer Differenz-Kalorimetrie (DSC) und mit dynamisch-mechanischer Thermoanalyse (DMTA) untersucht. DSC-Messungen wurden auf einem Netzsch DSC 204 Phoenix-Gerät durchgeführt. Dafür wurden 5 bis 10 mg der Proben in einem Aluminium-Gefäß eingewogen und die Messungen unter Stickstoffatmosphäre mit einer Abkühl- und Heizrate von 1 K·min⁻¹ in einem Temperaturbereich von -100 und +100°C durchgeführt. Die Ergebnisse sind in Tabelle 2 zusammengefasst. DMTA-Messungen wurden auf einem Eplexor 5 N (Gabo) ausgeführt, welches mit einem 25 N-Kraftaufnehmer ausgestattet war. Die statische Last betrug 0,50%, die dynamische Last 0,20%, die Frequenz 10 Hz und die Heizrate 2 K·min⁻¹ in einem Temperaturbereich von -100 und +100°C. Die Ergebnisse sind ebenfalls in Tabelle 2 zusammengefasst. Figur 2 zeigt für das Polymernetzwerk PCL(50)PEG den mit DSC gemessenen Wärmefluss dQ/dt (Einheit W/g) sowie das mit DMTA ermittelte Speichermodul E' (Einheit MPa) im Temperaturbereich von 0 bis 80°C.

**Tabelle 2**

| | DSC | | | DMTA | | |
|---|---|---|---|---|---|---|
| Polymer^{#} | T_{g} [°C] | Tₘ (PEG) [°C] | Tₘ (PCL) [°C] | T_{g} [°C] | Tₘ (PEG) [°C] | Tₘ (PCL) [°C] |
| P[PEGMMA] | n.b. | 39,3 ± 0,5 | - | n.b. | n.b. | n.b. |
| PCL(20)PEG | n.b. | 38,3 ± 0,5 | 53,3 ± 0,5 | n.b. | n.b. | n.b. |
| PCL(30)PEG | -64,1 ± 1,0 | 37,7 ± 0,5 | 54,5 ± 0,5 | -53 ± 1 | 36 ± 1 | 53 ± 1 |
| PCL(40)PEG | -60,9 ± 1,0 | 37,3 ± 0,5 | 53,2 ± 0,5 | -54 ± 1 | 38 ± 1 | 52 ± 1 |
| PCL(50)PEG | -61,6 ± 1,0 | 35,9 ± 0,5 | 52.7 ± 0,5 | -55 ± 1 | 36 ± 1 | 55 ± 1 |
| PCL(60)PEG | -60,9 ± 1,0 | 32,8 ± 0,5 | 54,5 ± 0,5 | -54 ± 1 | 37 ± 1 | 50 ± 1 |
| PCL(70)PEG | -61,4 ± 1,0 | 25,5 ± 0,5 | 53,0 ± 0,5 | -56 ± 1 | 7 ± 1 | 51 ± 1 |
| PCL(80)PEG | -63,6 ± 1,0 | 17,4 ± 0,5 | 53,4 ± 0,5 | -58 ± 1 | 3 ± 1 | 52 ± 1 |
| PCL(100) | -60,8 ± 1,0 | - | 55,7 ± 0,5 | -53 ± 1 | - | 54 ± 1 |

| | | | | | | |
|---|---|---|---|---|---|---|
| ^{#} Die in den Klammern angegebenen Zahlen bezeichnen den Massenanteil an PCL10kDMA im Polymemetzwerk. | | | | | | |

Es ist ersichtlich, dass das erfindungsgemäße, PCL- und PEG-Segmente enthaltende Polymernetzwerk im Bereich zwischen 0 und 80°C zwei gut differenzierte Phasenübergänge aufweist, die sich auf das Schmelzen von PEG- und PCL-Kristalliten zurückführen lassen. Dabei ist die untere Schmelztemperatur Tₘ eindeutig mit dem Schmelzen bzw. der Kristallisation von PEG-Segmenten assoziiert, die in dem Homopolymer P[PEGMMA] bei 39°C liegt und in den Copolymeren mit einem PCL-Massenanteil zwischen 20 und 60% bei 38 bis 32°C beobachtet wird. Die obere Schmelztemperatur Tₘ kann hingegen eindeutig dem Schmelzen bzw. der Kristallisation von PCL-Segmenten zugeordnet werden, die in dem Homopolymer PCL(100) bei etwa 55°C liegt und bei sämtlichen Copolymeren bei 53 bis 54°C beobachtet wird. Diese Ergebnisse zeigen, dass das erfindungsgemäße Pfropfpolymernetzwerk eine phasenseparierte Morphologie aufweist, in welcher die PCL- und PEG-Segmente eigene Domänen mit eigenen Übergangstemperaturen ausbilden, die zur temperaturgesteuerten Fixierung zweier temporären Formen geeignet sind.

Die Beobachtung, wonach die Schmelztemperatur der PEG-Segmente ab einem PCL-Anteil von 70% stark abfällt, zeigt, dass ein kritischer PEG-Massenanteil von 40% im Polymernetzwerk erforderlich ist, um eine geeignete PEG-Kristallgröße zu erhalten. Demgegenüber kann aus der konstanten Schmelztemperatur der PCL-Segmente geschlossen werden, dass bei einem PCL-Massenanteil von 20% bereits die erforderliche kritische Kristallgröße der PCL-Segmente erreicht ist.

### 4. Programmierung eines Polymernetzwerkes aus PCLDMA und PEGMMA

Ein Pfropfpolymernetzwerk PCL(40)PEG hergestellt nach Beispiel 2 basierend auf 40 Gew.-% PCL10kDMA und 60 Gew.-% PEG1kMMA wurde in einem zyklischen thermomechanischen Experiment derart programmiert, dass neben der herstellungsbedingten permanenten Form zwei temporäre Formen in dem "Formgedächtnis" des Polymers gespeichert wurden. Dies erfolgt prinzipiell durch Fixierung einer ersten temporären Form bei einer Temperatur unterhalb der Schmelztemperatur von PCL (Tₘ(PCL)) oder einer Temperatur unterhalb der Schmelztemperatur von PEG (Tₘ(PEG)) und anschließendem Fixieren einer zweiten temporären Form bei einer Temperatur unterhalb der Schmelztemperatur von PEG (Tₘ(PEG)).

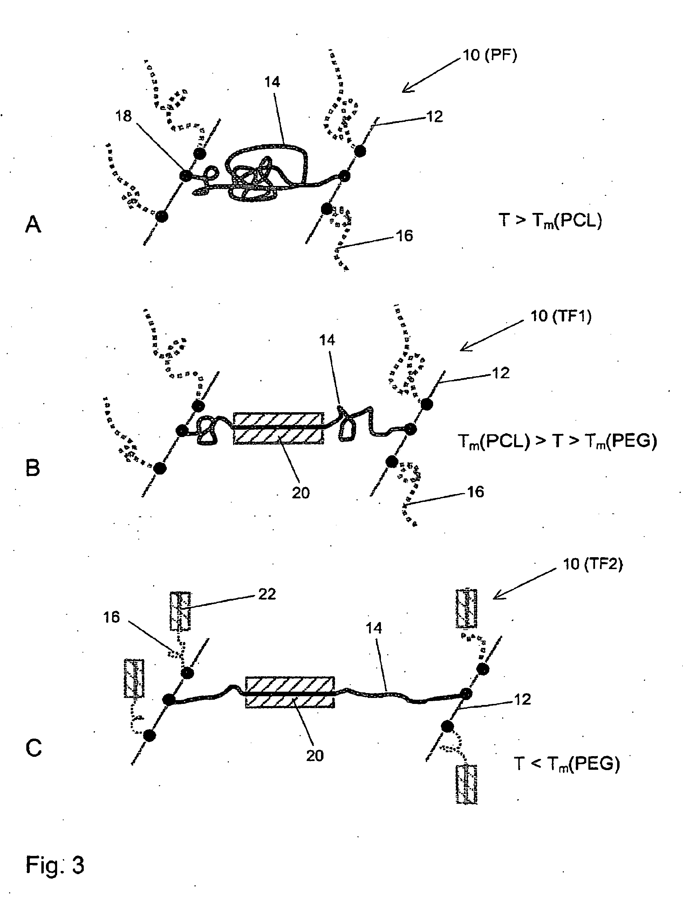

Dieses Prinzip ist anhand von Figur 3 erläutert, wobei die gleichen Bezugszeichen wie in Figur 2 verwendet werden. Dabei zeigt Figur 3A die Struktur des Polymernetzwerkes 10 oberhalb der oberen Übergangstemperatur, das heißt oberhalb der Schmelztemperatur Tₘ(PCL) der PCL-Segmente 14. Bei dieser Temperatur liegen sowohl die PCL-Segmente 14 als auch die PEG-Segmente 16 in amorphem Zustand vor, was zeichnerisch durch ungeordnete Segmente 14, 16 dargestellt ist. Das Polymer 10 liegt in dieser Startphase des Programmierverfahrens zunächst noch in seiner permanenten Form PF vor, die durch den Herstellungsprozess bedingt ist, insbesondere durch eine während der Vernetzung vorgegebene Form.

Ausgehend von der in Figur 3A gezeigten Form wird in einem ersten Schritt das Polymernetzwerk 10 in eine Form gebracht, welche einer ersten temporären Form TF1 entspricht. Dies erfolgt durch Ausübung einer geeigneten mechanischen Belastung oberhalb von Tₘ(PCL), die beispielweise zu einer Elongation des Polymers 10 führt. Dies ist in Figur 3B durch einen vergrößerten Abstand der Rückgratketten 12 angedeutet. Nach der Elongation wird das Polymersystem 10 auf eine Temperatur abgekühlt, die in jedem Fall unterhalb der Schmelztemperatur Tₘ(PCL) liegt, insbesondere zwischen Tₘ(PEG) und Tₘ(PCL). Die Abkühlung führt zu einer zumindest abschnittsweisen Kristallisation der PCL-Segmente 14. Die kristallinen PCL-Segmente sind in Figur 3B mit 20 bezeichnet. Die erste temporäre Form TF1 kann optional durch Tempern bei der T < Tₘ(PCL) für eine vorbestimmte Dauer stabilisiert werden. Die mechanische Belastung wird währenddessen aufrechterhalten.

Im nächsten Schritt erfolgt die Programmierung der zweiten temporären Form TF2 analog zur ersten temporären Form TF1. Insbesondere wird durch einen zweiten mechanischen Stimulus das Polymer 10 in die zweite temporäre Form TF2 überfährt, was beispielsweise durch eine weitere Elongation bei einer Temperatur oberhalb von Tₘ(PEG) erfolgen kann (Figur 3C). Anschließend wird auf eine Temperatur unterhalb der unteren Übergangstemperatur, das heißt der Schmelztemperatur Tₘ(PEG) der PEG-Segmente abgekühlt, um auch die zweite temporäre Form TF2 zu fixieren. Dabei kommt es zur Ausbildung von kristallinen PEG-Segmenten 22 der PEG-Seitenketten 16. Unter Aufrechterhaltung der mechanischen Belastung kann auch in dieser Stufe das Polymernetzwerk 10 noch für eine gewisse Zeit getempert werden, wodurch auch die Bildung von PCL-Kristalliten gefördert wird.

Ausgehend von einem auf diese Weise programmierten Polymernetzwerk 10, das in seiner zweiten temporären Form TF2 vorliegt, können die erste temporäre Form TP1 und die permanente Form PF nacheinander abgerufen werden, wenn das Polymer 10 erst auf eine Zwischentemperatur Tₘ(PEG) < T < Tₘ(PCL) und anschließend auf eine Temperatur oberhalb von Tₘ(PCL) erwärmt wird. Das Wiederherstellen zuvor fixierter Formen wird als Formgedächtnis- oder Shape-Memory-Effekt (SM-Effekt) bezeichnet.

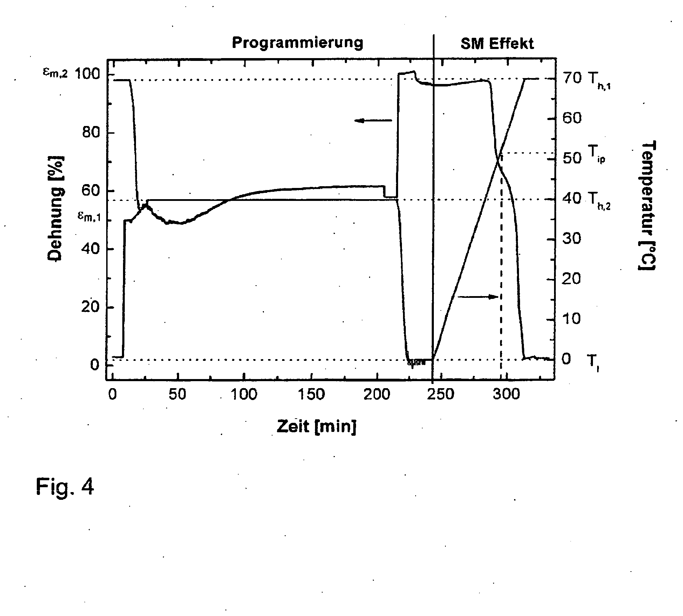

Figur 4 stellt die Verläufe der Temperatur sowie der Dehnung während eines Programmierungszyklus und Wiederabrufungszyklus des Polymers PCL(40)PEG dar.

Der Programmierungszyklus startet bei einer Temperatur T_{h,1} von 70°C oberhalb Tₘ(PCL) (etwa 53°C). Es erfolgt eine Dehnung des Polymers auf 50% (ε_{m,1}) entsprechend der ersten temporären Form TF1. Anschließend wird unter Aufrechterhaltung der mechanischen Last mit einem Temperaturgradienten von 4 K·min⁻¹ auf eine Zwischentemperatur von 40°C (T_{h,2}) unterhalb Tₘ(PCL) und oberhalb Tₘ(PEG) (etwa 37°C) abgekühlt. Nach einer Haltezeit von 3 h wird das Polymer entspannt, wobei ein leichter Rückgang der Ausdehnung beobachtet wird. Anschließend wird die Probe noch für 10 min ohne mechanische Last gehalten, um sie dann auf 100 % Gesamtausdehnung zu dehnen (ε_{m,2}), unter konstanter mechanischer Last auf 0°C (T_{I}) abzukühlen und für weitere 10 min die mechanische Last aufrechtzuerhalten, wobei die Dehnung etwas abnimmt.

Nach Beendigung des Programmierungszyklus erfolgt nacheinander der Abruf der gespeicherten Formen, indem (ohne mechanische Last) mit einer Aufheizrate von 1 K·min⁻¹ die Probe von 0 auf 70°C wieder erwärmt wird. Dabei wird zunächst das Aufschmelzen der PEG-Kristallite und die Wiederherstellung der zweiten temporären Form um Tₘ(PEG) beobachtet. Wird die Temperatur für eine Stunde bei 40°C gehalten, bleibt die zweite temporäre Form stabil und es erfolgt kein Übergang in die permanente Form (nicht dargestellt).

Die weitere Aufheizung oberhalb Tₘ(PCL) führt zum Schmelzen der PCL-Kristallite und zur nahezu quantitativen Wiederherstellung der permanenten Form. Diese Programmierungs- und Wiederherstellungszyklus wurde weitere vier Mal mit dem gleichen Ergebnis durchgeführt.

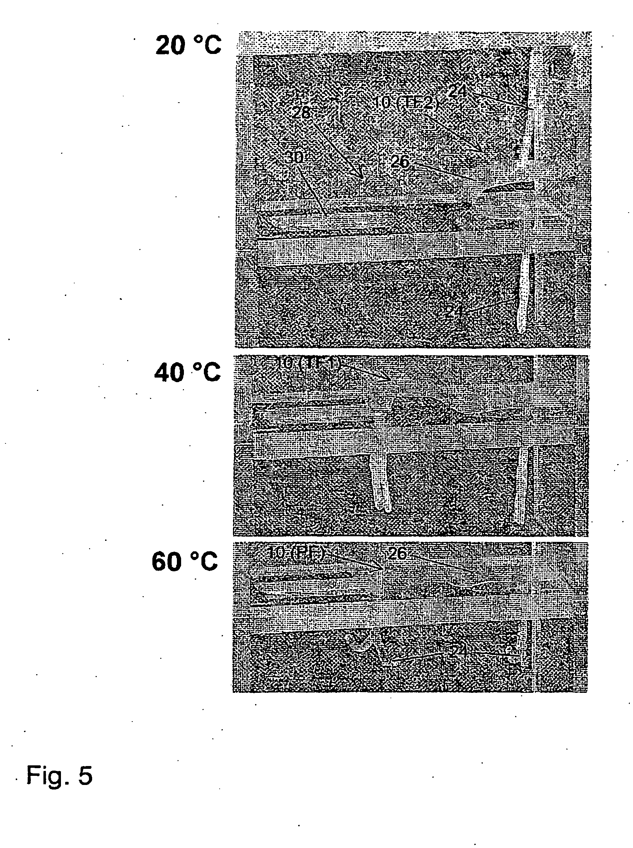

Ein Demonstrationsbeispiel für eine praktische Anwendung eines programmierten erfindungsgemäßen Polymernetzwerkes entsprechend Beispiel 2 ist in Figur 5 dargestellt. Dabei ist im oberen Teil der Abbildung die zweite temporäre Form TF2 des Polymers 10 bei einer Temperatur von 20°C gezeigt. Das Polymer 10 weist zwei längliche Seitenabschnitte 24 auf, die an einem flächigen Zentralabschnitt 26 befestigt sind. Das Polymer 10 ist auf einem transparenten Kunststoffträger 28 befestigt, der eine Öffnung 30 aufweist. Unter Erwärmung des Polymersystems 10 auf eine Temperatur von 40°C erfolgt eine Deformation des Zentralabschnitts 26 von der im oberen Teil dargestellten gebogenen Form in eine glatte Form (Fig. 5, mittlerer Teil), welche der ersten temporären Form TF1 entspricht. Dabei bleiben die Seitenabschnitte 24 weitestgehend unverändert. Während der Wiederherstellung der ersten temporären Form TF1 greift der linke Seitenabschnitt 24 durch die Öffnung 30 des Kunststoffträgers 28. Bei weiterer Erwärmung des Polymersystems 10 auf 60°C kommt es zu einem Aufbiegen der Seitenabschnitte 24, die nun eine hakenförmige Gestalt annehmen (Fig. 5, unterer Teil). Gleichzeitig bleibt der Zentralabschnitt 26 praktisch unverändert. Die im unteren Teil der Figur 5 gezeigte Form entspricht der permanenten Form PF des Polymernetzwerkes 10.

### BEZUGSZEICHENLISTE

- PF: permanente Form
- TF1: erste temporäre Form
- TF2: zweite temporäre Form
- T_{trans,1}: erste Übergangstemperatur
- T_{trans,2}: zweite Übergangstemperatur
- Tₘ(PCL): Schmelztemperatur der PCL-Segmente
- Tₘ(PEG): Schmelztemperatur der PEG-Segmente

- 10: Polymernetzwerk
- 12: Rückgrat
- 14: Vernetzendes Polymer (PCL10kDMA)
- 16: Seitenkettenpolymer (PEG1kMMA)
- 18: Verknüpfungspunkte
- 20: kristallines PCL-Segment
- 22: kristallines PEG-Segment
- 24: Seitenabschnitt
- 26: Zentralabschnitt
- 28: Kunststoffträger
- 30: Öffnung

## Patentansprüche

1. Formgedächtnispolymer, das mindestens zwei Schaltsegmente mit unterschiedlichen Übergangstemperaturen (T_{trans,1}, T_{trans,2}) aufweist, sodass das Polymer in Abhängigkeit von der Temperatur neben einer permanenten Form (PF) mindestens zwei temporäre Formen (TF1, TF2) einnehmen kann, wobei ein erstes Schaltsegment im Wesentlichen auf einem Polyester der allgemeinen Formel I mit n = 1...6 oder einem Co-Polyester der allgemeinen Formel I mit unterschiedlichen n oder einem Derivat von diesen und ein zweites Schaltsegment im Wesentlichen auf einem Polyether der allgemeinen Formel II mit m = 1 ...4 oder einem Co-Polyether der allgemeinen Formel II mit unterschiedlichen m oder einem Derivat von diesen basiert 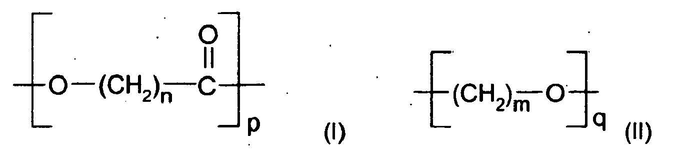 **dadurch gekennzeichnet, dass**
eines der Schaltsegmente vernetzt vorliegt und das andere Schaltsegment in Form freier, an dem vernetzten Schaltsegment öder an einer polymeren Rückgratstrukuür gebunden Seitenketten vorliegt.

2. Formgedächtnispolymer nach Anspruch 1,
**dadurch gekennzeichnet, dass**
das erste Schaltsegment ein Poly(ε-caprolacton)-Segment mit n = 5 oder ein Derivat von diesem umfasst, in dem die aliphatischen Kohlenstoffatome unabhängig voneinander mit einem oder zwei, unverzweigten oder verzweigten, gesättigten oder ungesättigten C1- bis C6-Resten substituiert sein können.

3. Formgedächtnispolymer nach einem der vorhergehenden Ansprüche,
**dadurch gekennzeichnet, dass**
das zweite Schaltsegment ein Polyethylenglykol-Segment mit m = 2 oder ein Derivat von diesem umfasst, in dem die aliphatischen Kohlenstoffatome unabhängig voneinander mit einem oder zwei, unverzweigten oder verzweigten, gesättigten oder ungesättigten C1- bis C6-Resten substituiert sein können.

4. Formgedächtnispolymer nach einem der vorhergehenden Ansprüche,
**dadurch gekennzeichnet, dass**
das erste Schaltsegment ein mittleres Molekulargewicht im Bereich von 2.000 bis 100.000 g/mol, insbesondere von 5.000 bis 40.000 g/mol, vorzugsweise von etwa 10.000 g/mol aufweist.

5. Formgedächtnispolymer nach einem der vorhergehenden Ansprüche,
**dadurch gekennzeichnet, dass**
das zweite Schaltsegment ein mittleres Molekulargewicht im Bereich von 100 bis 10.000 g/mol, insbesondere von 500 bis 5.000 g/mol, vorzugsweise von etwa 1.000 g/mol aufweist.

6. Formgedächtnispolymer nach einem der vorhergehenden Ansprüche,
**dadurch gekennzeichnet, dass**
ein Massenanteil des Polyestersegments im Formgedächtnispolymer im Bereich von 25 bis 65 %, insbesondere im Bereich von 30 bis 60 %, beträgt.

7. Formgedächtnispolymer nach einem der vorhergehenden Ansprüche,
**dadurch gekennzeichnet, dass**
der Polyester vernetzt vorliegt und der Polyether als freie Seitenketten an dem vernetzten Schaltsegment oder an der polymeren Rückgratstruktur gebunden ist.

8. Formgedächtnispolymer nach einem der vorhergehenden Ansprüche,
**dadurch gekennzeichnet, dass**
die polymere Rückgratstruktur durch Polyacrylat- oder Polymethacrylatketten gebildet ist.

9. Verfahren zur Herstellung eines Formgedächtnispolymers nach einem der Ansprüche 1 bis 8, wobei ein Polyester der allgemeinen Formel I a mit n = 1...6 und Y ein verbindender Rest ist oder einem Co-Polyester der allgemeinen Formel I a mit unterschiedlichen n oder einem Derivat von diesen und ein Polyether der allgemeinen Formel II a mit m = 1...4 oder einem Co-Polyether der allgemeinen Formel II mit unterschiedlichen m oder einem Derivat von diesen copolymerisiert werden,

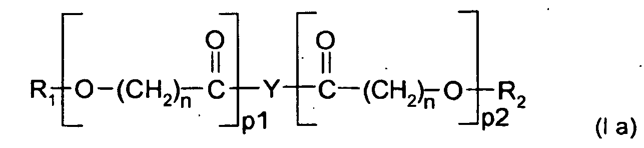

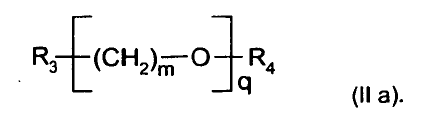

10. Verfahren nach Anspruch .9,
**dadurch gekennzeichnet, dass**
die erste Endgruppe R₁ und/oder die zweite Endgruppe R₂ des ersten Schaltsegments unabhängig voneinander ein polymerisierbarer Rest, insbesondere R₁ und R₂ jeweils ein polymerisierbarer Rest sind.

11. Verfahren nach Anspruch 10,
**dadurch gekennzeichnet, dass**
die erste Endgruppe R₁ und/oder die zweite Endgruppe R₂ des ersten Schaltsegments ein Acryl- oder Methacrylrest sind, insbesondere jeweils ein Methacrylrest sind.

12. Verfahren nach einem der Ansprüche 9 bis 11,
**dadurch gekennzeichnet, dass**
die erste Endgruppe R₃ und/oder die zweite Endgruppe R₄ des zweiten Schaltsegments unabhängig voneinander ein polymerisierbarer Rest sind, insbesondere nur eine der Endgruppen R₃ oder R₄ ein polymerisierbarer Rest ist und die andere ein nicht reaktiver Rest.

13. Verfahren nach Anspruch 12,
**dadurch gekennzeichnet, dass**
die erste Endgruppe R₃ oder die zweite Endgruppe R₄ ein Acryl- oder ein Methacrylrest ist und die andere Endgruppe ein Alkoxyrest, wobei insbesondere die erste Endgruppe R₃ ein Methyletherrest und die zweite Endgruppe R₄ ein Methacrylatrest ist.

14. Verfahren zur Programmierung von mindestens zwei temporären Form (TF1, TF2) bei einem Formgedächtnispolymer nach einem der Ansprüche 1 bis 8, mit den Schritten
(a) Überführung des Formgedächtnispolymers in eine der ersten temporären Form (TF1) entsprechende Form bei einer Temperatur oberhalb der oberen Übergangstemperatur (T_{trans,1}),
(b) Abkühlung auf eine Temperatur unterhalb der oberen Übergangstemperatur (T_{trans,1}) unter Fixierung der ersten temporären Form (TF1),
(c) Überführung des Formgedächtnispotymers in eine der zweiten temporären Form (TF2) entsprechende Form bei einer Temperatur oberhalb der unteren Übergangstemperatur (T_{trans,2}) und unterhalb der oberen Übergangstemperatur (T_{trans,1}) und
(d) Abkühlung auf eine Temperatur unterhalb der unteren Übergangstemperatur (T_{trans,2}) unter Fixierung der zweiten temporären Form (TF2).

15. Verfahren nach Anspruch 14,
**dadurch gekennzeichnet, dass**
in Schritt (b) auf eine Temperatur unterhalb der oberen Übergangstemperatur (T_{trans,1}) und oberhalb der unteren Übergangstemperatur (T_{trans,2}) abgekühlt wird oder auf eine Temperatur unterhalb unteren Übergangstemperatur (T_{trans,2}).

16. Verwendung eines nach einem der Ansprüche 1 bis 8 als medizinisches Material, insbesondere Implantatmaterial.

## Claims

1. Shape memory polymer comprising at least two switch segments having different transition temperatures (T_{trans,1}, T_{trans,2}) so that the polymer may assume, as a function of temperature, at least two temporary shapes (TF1, TF2) as well as a permanent shape (PF), wherein a first switch segment is based essentially on a polyester of the general formula I where n = 1...6 or a copolyester of the general formula I having different n's or a derivative of these and a second switch segment is based essentially on a polyether of the general formula II where m = 1...4 or a copolyether of the general formula II having different m's or a derivative of these 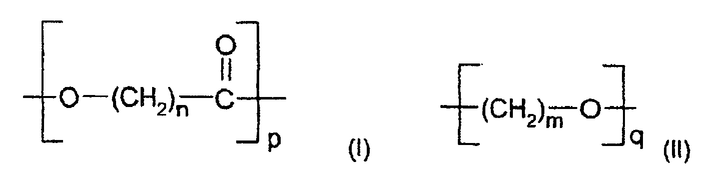 **characterized in that** one of the switch segments is present in a crosslinked state and the other switch segment is present in the form of free side chains attached to the crosslinked switch segment or to a polymeric backbone structure.

2. Shape memory polymer according to Claim 1, **characterized in that** the first switch segment comprises a poly(ε-caprolactone) segment where n = 5 or a derivative thereof in which the aliphatic carbon atoms, independently of each other, may be substituted with one or two, branched or unbranched, saturated or unsaturated C1 to C6 radicals.

3. Shape memory polymer according to either of the preceding claims, **characterized in that** the second switch segment comprises a polyethylene glycol segment where m = 2 or a derivative thereof in which the aliphatic carbon atoms, independently of each other, may be substituted with one or two, branched or unbranched, saturated or unsaturated C1 to C6 radicals.

4. Shape memory polymer according to any one of the preceding claims, **characterized in that** the first switch segment has an average molecular weight in the range from 2000 to 100 000 g/mol, particularly from 5000 to 40 000 g/mol, preferably of about 10 000 g/mol.

5. Shape memory polymer according to any one of the preceding claims, **characterized in that** the second switch segment has an average molecular weight in the range from 100 to 10 000 g/mol, particularly from 500 to 5000 g/mol, preferably of about 1000 g/mol.

6. Shape memory polymer according to any one of the preceding claims, **characterized in that** a mass fraction of the polyester segment in the shape memory polymer is in the range from 25% to 65%, particularly in the range from 30% to 60%.

7. Shape memory polymer according to any one of the preceding claims, **characterized in that** the polyester is present in the crosslinked state and the polyether is attached as free side chains to the crosslinked switch segment or to the polymeric backbone structure.

8. Shape memory polymer according to any one of the preceding claims, **characterized in that** the polymeric backbone structure is formed by polyacrylate or polymethacrylate chains.

9. Method of preparing a shape memory polymer according to any one of Claims 1 to 8, which comprises a polyester of the general formula I a where n = 1...6 and Y is a linking radical or a copolyester of the general formula I a having different n's or a derivative of these and a polyether of the general formula II a where m = 1...4 or a copolyether of the general formula II having different m's or a derivative of these being copolymerized

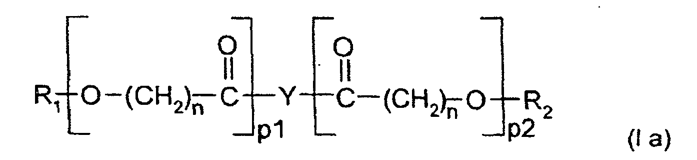

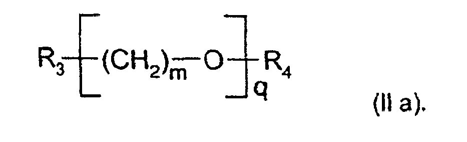

10. Method according to Claim 9, **characterized in that** the first end group R₁ and/or the second end group R₂ or the first switch segment, independently of each other, are a polymerizable radical, more particularly R₁ and R₂ are each a polymerizable radical.

11. Method according to Claim 10, **characterized in that** the first end group R₁ and/or the second end group R₂ of the first switch segment are an acryloyl or methacryloyl radical, more particularly are each a methacryloyl radical.

12. Method according to any one of Claims 9 to 11, **characterized in that** the first end group R₃ and/or the second end group R₄ of the second switch segment, independently of each other, are a polymerizable radical, more particularly only one of the end groups R₃ or R₄ is a polymerizable radical and the other is a non-reactive radical.

13. Method according to Claim 12, **characterized in that** the first end group R₃ and/or the second end group R₄ is an acryloyl or a methacryloyl radical and the other end group is an alkoxy radical, more particularly the first end group R₃ is a methyl ether radical and the second end group R₄ is a methacrylate radical.

14. Method of programming at least two temporary shapes (TF1, TF2) in a shape memory polymer according to any one of Claims 1 to 8, comprising the steps
(a) converting the shape memory polymer at a temperature above the upper transition temperature (T_{trans,1}) into a shape corresponding to the first temporary shape (TF1),
(b) cooling to a temperature below the upper transition temperature (T_{trans,1}) to fix the first temporary shape (TF1),
(c) converting the shape memory polymer at a temperature above the lower transition temperature (T_{trans,2}) and below the upper transition temperature (T_{trans,1}) into a shape corresponding to the second temporary shape (TF2), and
(d) cooling to a temperature below the lower transition temperature (T_{trans,2}) to fix the second temporary shape (TF2).

15. Method according to Claim 14, **characterized in that** the cooling in step (b) is to a temperature below the upper transition temperature (T_{trans,1}) and above the lower transition temperature (T_{trans,2}) or to a temperature below the lower transition temperature (T_{trans,2}).

16. Use of a shape memory polymer according to any one of Claims 1 to 8 as a medical material, more particularly implant material.

## Revendications

1. Polymère à mémoire de forme, qui présente au moins deux segments commutés avec des températures de transition différentes (T_{trans,1} T_{trans,2}), de manière telle que le polymère, en fonction de la température, peut prendre au moins deux formes temporaires (TF1, TF2), en plus d'une forme permanente (PF), où un premier segment de commutation est essentiellement à base d'un polyester de formule générale I avec n = 1...6 ou d'un copolyester de formule générale I avec différents n ou d'un dérivé de celui-ci et un deuxième segment de commutation est essentiellement à base d'un polyéther de formule générale II avec m = 1...4 ou d'un copolyéther de formule générale II avec différents m ou d'un dérivé de celui-ci 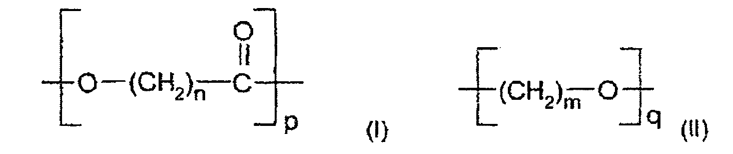 **caractérisé en ce qu'**un des segments commutés se trouve sous forme réticulée et l'autre segment commuté se trouve sous forme de chaînes latérales libres, liées au segment commuté réticulé ou liées à une structure d'épine dorsale polymère.

2. Polymère à mémoire de forme selon la revendication 1, **caractérisé en ce que** le premier segment commuté comprend un segment de type poly(ε-caprolactone) avec n = 5 ou un dérivé de celui-ci, dans lequel les atomes de carbone aliphatiques peuvent être substitués, indépendamment l'un de l'autre, par un ou deux radicaux en C₁-C₆ non ramifiés ou ramifiés, saturés ou insaturés.

3. Polymère à mémoire de forme selon l'une quelconque des revendications précédentes, **caractérisé en ce que** le deuxième segment commuté comprend un segment de type polyéthylèneglycol avec m = 2 ou un dérivé de celui-ci, dans lequel les atomes de carbone aliphatiques peuvent être substitués, indépendamment l'un de l'autre, par un ou deux radicaux en C₁-C₆ non ramifiés ou ramifiés, saturés ou insaturés.

4. Polymère à mémoire de forme selon l'une quelconque des revendications précédentes, **caractérisé en ce que** le premier segment commuté présente un poids moléculaire moyen dans la plage de 2000 à 100 000 g/mole, en particulier de 5000 à 40 000 g/mole, de préférence d'environ 10 000 g/mole.

5. Polymère à mémoire de forme selon l'une quelconque des revendications précédentes, **caractérisé en ce que** le deuxième segment commuté présente un poids moléculaire moyen dans la plage de 100 à 10 000 g/mole, en particulier de 500 à 5000 g/mole, de préférence d'environ 1000 g/mole.

6. Polymère à mémoire de forme selon l'une quelconque des revendications précédentes, **caractérisé en ce qu'**une proportion massique du segment de polyester dans le polymère à mémoire de forme se situe dans la plage de 25 à 65%, en particulier dans la plage de 30 à 60%.

7. Polymère à mémoire de forme selon l'une quelconque des revendications précédentes, **caractérisé en ce que** le polyester se trouve sous forme réticulée et le polyéther est lié sous forme de chaînes libres au segment commuté réticulé ou à la structure d'épine dorsale polymère.

8. Polymère à mémoire de forme selon l'une quelconque des revendications précédentes, **caractérisé en ce que** la structure d'épine dorsale polymère est formée par des chaînes de polyacrylate ou de polyméthacrylate.

9. Procédé pour la préparation d'un polymère à mémoire de forme selon l'une quelconque des revendications 1 à 8, où un polyester de formule générale Ia avec n = 1...6 et Y, représentant un radical de liaison, ou un copolyester de formule générale Ia avec différents n ou un dérivé de celui-ci et un polyéther de formule générale IIa avec m = 1...4 ou un copolyéther de formule générale II avec différents m ou un dérivé de celui-ci sont copolymérisés.

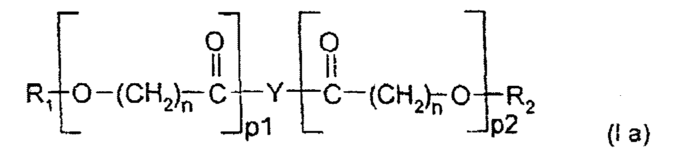

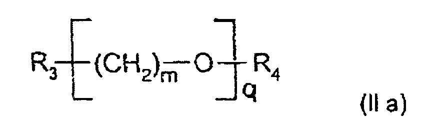

10. Procédé selon la revendication 9, **caractérisé en ce que** le premier groupe terminal R₁ et/ou le deuxième groupe terminal R₂ du premier segment commuté représente(nt) indépendamment l'un de l'autre un radical polymérisable, en particulier R₁ et R₂ représentent chacun un radical polymérisable.

11. Procédé selon la revendication 10, **caractérisé en ce que** le premier groupe terminal R₁ et/ou le deuxième groupe terminal R₂ du premier segment commuté représente(nt) un radical acryle ou méthacryle, en particulier chacun un radical méthacryle.

12. Procédé selon l'une quelconque des revendications 9 à 11, **caractérisé en ce que** le premier groupe terminal R₃ et/ou le deuxième groupe terminal R₄ du deuxième segment commuté représente(nt) indépendamment l'un de l'autre un radical polymérisable, en particulier seulement un des groupes terminaux R₃ ou R₄ représente un radical polymérisable et l'autre représente un radical non réactif.

13. Procédé selon la revendication 12, **caractérisé en ce que** le premier groupe terminal R₃ ou le deuxième groupe terminal R₄ représente un radical acryle ou un radical méthacryle et l'autre groupe terminal représente un radical alcoxy, le premier groupe terminal R₃ étant en particulier un radical méthyléther et le deuxième groupe terminal R₄ étant en particulier un radical méthacrylate.

14. Procédé pour la programmation d'au moins deux formes temporaires (TF1, TF2) d'un polymère à mémoire de forme selon l'une quelconque des revendications 1 à 8, présentant les étapes
(a) transformation du polymère à mémoire de forme dans une forme correspondant à la première forme temporaire (TF1) à une température au-dessus de la température de transition supérieure (T_{trans,1})
(b) refroidissement à une température au-dessous de la température de transition supérieure (T_{trans,1}) avec fixation de la première forme temporaire (TF1),
(c) transformation du polymère à mémoire de forme dans une forme correspondant à la deuxième forme temporaire (TF2) à une température au-dessus de la température de transition inférieure (T_{trans,2}) et au-dessous de la température de transition supérieure (T_{trans,1}) et
(d) refroidissement à une température au-dessous de la température de transition inférieure (T_{trans,2}) avec fixation de la deuxième forme temporaire (TF2).

15. Procédé selon la revendication 14, **caractérisé en ce qu'**on refroidit dans l'étape (b) à une température au-dessous de la température de transition supérieure (T_{trans,1}) et au-dessus de la température de transition inférieure (T_{trans,2}) ou à une température au-dessous de la température de transition inférieure (T_{trans,2}).

16. Utilisation d'un polymère à mémoire de forme selon l'une quelconque des revendications 1 à 8 comme matériau médical, en particulier matériau d'implantation.
